(19) Europäisches Patentamt
European Patent Office
Office européen des brevets

(11) Veröffentlichungsnummer: **0 092 100**
**B1**

# (12) EUROPÄISCHE PATENTSCHRIFT

(45) Veröffentlichungstag der Patentschrift: **18.12.85**

(51) Int. Cl.⁴: **C 07 D 405/06**, A 01 N 43/64

(21) Anmeldenummer: **83103312.1**

(22) Anmeldetag: **05.04.83**

(54) **Triazolylalkyl-2,3-dihydrobenzofuran-oxime, Verfahren zu ihrer Herstellung und ihre Verwendung als Pflanzenwachstumsregulatoren.**

(30) Priorität: **10.04.82 DE 3213373**

(43) Veröffentlichungstag der Anmeldung: **26.10.83 Patentblatt 83/43**

(45) Bekanntmachung des Hinweises auf die Patenterteilung: **18.12.85 Patentblatt 85/51**

(84) Benannte Vertragsstaaten: **AT BE CH DE FR GB IT LI NL**

(56) Entgegenhaltungen: **EP - A - 0 056 089**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Reissenweber, Gernot, Dr., Drosselstrasse 15, D-6737 Boehl-Iggelheim (DE)**
Erfinder: **Feuerherd, Karl-Heinz, Dr., 25-11, Higashi Yukigaya 4-chome, Ohta-ku Tokyo 145 (JP)**
Erfinder: **Jung, Johann, Dipl.-Landwirt, Dr., Hardenburgstrasse 19, D-6703 Limburgerhof (DE)**

**EP 0 092 100 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue Triazolylalkyl-2,3-dihydrobenzofuran-oxime, Verfahren zu ihrer Herstellung, Mittel zur Regulierung des Pflanzenwachstums, die diese Verbindungen enthalten, und Verfahren zur Regulierung des Pflanzenwachstums mit diesen Verbindungen.

Es ist bereits bekannt, das 2-Chlorethyltrimethylammoniumchlorid (Chlorcholinchlorid, CCC) (J. Biol. Chem., *235*, 475 (1960)) zur Beeinflussung des Pflanzenwachstums zu verwenden. Mit seiner Hilfe lässt sich z.B. eine Hemmung des Längenwachstums bei einigen Getreidearten und eine Hemmung des vegetativen Wachstums bei einigen anderen Kulturpflanzen erreichen. Die Wirkung dieses Stoffes ist jedoch insbesondere bei niedrigen Aufwandmengen, nicht immer ausreichend.

Es ist ferner aus der DE-OS 2650831 bekannt, 1-(4-Bromphenyl)-1-allyloxy-2-(1,2,4-triazolyl-(1))-ethan zur Regulierung des Pflanzenwachstums von Raps, Weizen, Roggen und Gerste zu verwenden. Bei niedrigen Aufwandmengen werden aber die in der Praxis gestellten Forderungen bezüglich der Wirksamkeit nicht immer in ausreichendem Masse erfüllt.

Es wurde nun gefunden, dass Verbindungen der Formel I

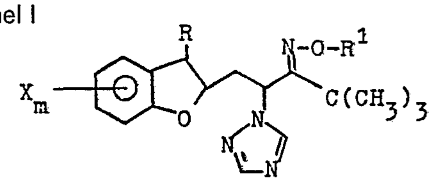
(I)

in der

R ein Wasserstoffatom oder einen Alkylrest mit bis zu 4 C-Atomen,

X ein Wasserstoff-, Fluor-, Chlor-, Brom- oder Iodatom, einen Nitro-, Trifluormethyl- oder einen Alkylrest mit bis zu 4 C-Atomen, einen Alkoxyrest mit bis zu 2 C-Atomen oder einen Alkenylrest mit 3 bis 4 C-Atomen, einen Phenyl- oder Phenoxyrest bedeutet und

m eine ganze Zahl von 1 bis 4 bedeutet, wobei die einzelnen Gruppen X gleich oder verschieden sind, wenn m grösser als 1 ist, in der ferner

$R^1$ ein Wasserstoffatom, einen gegebenenfalls chlorsubstitutierten Alkylrest mit 1 bis 6 C-Atomen oder einen gegebenenfalls chlorsubstituierten Alkenylrest mit 3 bis 5 C-Atomen, einen Alkinylrest mit 3 bis 4 C-Atomen oder einen Benzylrest bedeutet, wobei der Benzylrest durch ein Fluor-, Chlor- oder Bromatom, eine Nitrogruppe, einen Trifluormethyl- oder einen Alkylrest mit bis zu 4 C-Atomen substituiert sein kann, oder in der $R^1$ einen $-CO-R^2-$Rest bedeutet, wobei

$R^2$ Methyl, Ethyl, n-Propyl, Isopropyl, Chlormethyl, Chlorpropyl, Methoxymethyl, Acetonyl ($CH_3-COCH_2-$), Vinyl oder Propenyl bedeutet, sowie deren Säureadditionssalze und Metalkomplexe hervorragend zur Beeinflussung des Pflanzenwachstums geeignet sind und eine sehr gute Pflanzenverträglichkeit besitzen.

Die neuen Verbindungen der Formel I enthalten chirale Zentren und werden im allgemeinen in Form von Racematen oder als Diastereomerengemische von erythro- sowie threo-Formen erhalten. Die erfindungsgemässen Verbindungen der Formel I können zusätzlich in zwei geometrischen Isomerenformen (Z und E) entstehen. Aus den bei der Synthese üblicherweise anfallenden Isomerengemischen kann man mit bekannten Methoden einheitliche geometrische Isomere bzw. einheitliche Enantiomere erhalten. Als Mittel zur Beeinflussung des Pflanzenwachstums kann man sowohl die einheitlichen geometrischen Isomeren bzw. Enantiomeren wie auch deren bei der Synthese anfallenden Gemische verwenden. Während aus wirtschaftlichen Gründen i.a. die Gemische bevorzugt werden, weisen die Stereoisomeren bzw. Enantiomeren vielfach eine besonders günstige spezifische Wirkung auf.

X bedeutet beispielsweise 5-Fluor, 6-Fluor, 5-Chlor, 6-Chlor, 5-Brom, 6-Brom, 5-Methyl, 7-Methyl, 5-Methoxy, 6-Methoxy, 5-Trifluormethyl, 6-Trifluormethyl, 5-tert.-Butyl, 6-tert.-Butyl, 5-Nitro, 6-Nitro, 4,5-Dichlor, 5,7-Dichlor, 5,6-Dichlor, 5-Brom-7-Chlor, 5-Chlor-7-brom, 5-Chlor-7-methyl, 5-Methyl-7-chlor, 4,5,7-Trichlor, 5,7-Dimethyl, 4,6-Dimethyl, 5-Ethoxy, 5-n-Butoxy und 5-Phenoxy.

R bedeutet beispielsweise Wasserstoff, Methyl, Ethyl, n-Propyl, Isopropyl und n-Butyl.

$R^1$ bedeutet beispielsweise Wasserstoff, Methyl, Ethyl, n-Propyl, n-Butyl, 4-Chlorbutyl, Allyl, 1-Buten-2-yl-propargyl, Benzyl, 4-Fluorbenzyl, 4-Chlorbenzyl, 4-Brombenzyl, 2,4-Dichlorbenzyl, 4-Methylbenzyl und 4-Trifluormethylbenzyl.

Geeignete Säureadditionssalze sind beispielsweise die Chloride, Bromide, Sulfate, Nitrate, Phosphate, Oxalate oder Dodecylsulfonate. Die Wirksamkeit der Salze geht auf das Kation zurück, so dass das Anion, soweit es pflanzenverträglich ist, beliebig gewählt werden kann. Metallkomplexsalze sind beispielsweise die Komplexe mit Kupferchlorid, Kupferacetat, Kobaltchlorid, Nickelchlorid, Eisenchlorid und Zinkacetat.

Die neuen Triazolylalkyl-2,3-dihydrobenzofuran-oxime der Formel I erhält man beispielsweise, wenn man Triazolylalkyl-2,3-dihydrobenzofuranone der Formel II

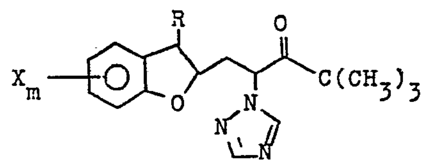
(II)

mit entsprechenden Hydroxylamin-Derivaten der Formel III

$$H_2N-O-R^1 \qquad (III)$$

vorzugsweise in Gegenwart eines basischen oder sauren Katalysators und gegebenenfalls in Gegenwart eines Verdünnungsmittels umsetzt.

Man kann auch ausgehen von Verbindungen der Formel I, in der $R^1$ ein Wasserstoffatom bedeutet, wie sie durch Umsetzung der Ketone II mit Hydroxylamin erhalten werden und die so erhaltenen unsubstituierten Oxime nunmehr mit entsprechenden Alkyl-, Alkenyl-, Alkinyl- und Benzylha-

logeniden der Formel $R^1$-Hal (Hal bedeutet vorzugsweise Chlor oder Brom), einem entsprechenden Säurechlorid oder -anhydrid der Formel $R^2-CO-Hal$ bzw. $R^2-CO-O-CO-R^2$, vorzugsweise in Gegenwart einer anorganischen Base, gegebenenfalls unter Zusatz eines Reaktionsbeschleunigers und/oder Phasentransferkatalysators und gegebenenfalls in Gegenwart eines Lösungs- oder Verdünnungsmittels umsetzen.

Die Verbindungen der Formel II sind aus der DE-OS 3049542 bekannt bzw. können nach den darin beschriebenen Verfahren leicht hergestellt werden.

Die Ketone II werden zweckmässig in Wasser oder in einem mit Wasser mischbaren Lösungsmittel mit den Hydroxylaminen III umgesetzt. Hierzu gehören beispielsweise Alkohole, wie Methanol, Ethanol, Propanol, Ethylenglykol, 2-Methoxyethanol; Ether wie Tetrahydrofuran und Dioxan; Amide wie Dimethylformamid, Diethylformamid, Dimethylacetamid, ferner N-Methylpyrolidon und Hexamethyl-phosphorsäuretriamid. Dabei ist es zweckmässig, eine Base wie beispielsweise Natriumhydroxid, Kaliumhydroxid, Bariumhydroxid, Natriumacetat, ein Amin wie Triethylamin, Piperidin, N-Methylpiperidin oder eine Mineralsäure wie Salzsäure oder Schwefelsäure oder eine Carbonsäure wie Ameinsensäure oder Essigsäure zuzusetzen. Wenn als Säure eine Carbonsäure verwendet wird, kann sie auch als Lösungs- bzw. Verdünnungsmittel dienen. Die Reaktion verläuft i.a. bei einer ausreichenden Temperatur unter 100°C.

Die Triazolylalkyl-2,3-dihydrobenzofuranoxime der Formel I ($R^1=H$) können ebenfalls in einem Lösungsmittel mit Alkylierungsmitteln bzw. Säurechloriden bzw. -anhydriden umgesetzt werden. Hierfür empfiehlt sich beispielsweise Acetonitril; Ether wie Diethyl-, Dipropyl-, Dibutyl- oder Methyl-tert.-butylether, Tetrahydrofuran, Dioxan und Dimethoxyethan; Ester wie Essigsäureethylester; Ketone wie Aceton und Methylethylketon; chlorierte Kohlenwasserstoffe wie Methylenchlorid, Chloroform, Tetrachlorkohlenstoff, Dichlorethan, 1,1,1-Trichlorethan, Tetrachlorethan, Chlorbenzol; Kohlenwasserstoffe wie Benzol, Toluol und Xylol. Dabei ist es zweckmässig eine Base wie beispielsweise Alkali- und Erdalakalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiummethylat, Natriumisopropylat oder Kalium-tert-butylat, Amine wie Triethylamin, N,N-Dimethylcyclohexylamin, Piperidin, N-Methylpiperidin oder Pyridin zuzusetzen.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin oder 4-Pyrrolidinopyridin, Kronenether wie 12-Krone-4, 14-Krone-5, 18-Krone-6, Dibenzo-18-krone-6 oder Dicyclohexano-18-krone-6 oder Azole wie Imidazol und 1,2,4-Triazol.

Als Phasentransferkatalysatoren verwendet man zweckmässig die hierfür üblichen quaternären Ammoniumsalze wie Tetrabutylammoniumchlorid, -bromid, -iodid oder -hydrogensulfat, Benzyltriethylammoniumchlorid, Methyl-trioctylammoniumchlorid und -bromid und Phosphoniumsalze wie Tetrabutylphosphoniumbromid und -iodid und Tetra-n-pentylphosphoniumbromid und -iodid.

Als Beispiele für die neuen erfindungsgemässen Verbindungen der Formel I seien im einzelnen genannt:

1-(2',3'-Dihydrobenzofuran-2'-yl)-2-(1,2,4-triazol-1-yl)-4,4-dimethylpentan-3-on-oxim,
1-(2',3'-Dihydrobenzofuran-2'-yl)-2-(1,2,4-triazol-1-yl)-4,4-dimethylpentan-3-on-O-methyloxim,
1-(2',3'-Dihydrobenzofuran-2'-yl)-2-(1,2,4-triazol-1-yl)-4,4-dimethylpentan-3-on-O-ethyloxim,
1-(2',3'-Dihydrobenzofuran-2'-yl)-2-(1,2,4-triazol-1-yl)-4,4-dimethylpentan-3-on-O-butyloxim,
1-(2',3'-Dihydrobenzofuran-2'-yl)-2-(1,2,4-triazol-1-yl)-4,4-dimethylpentan-3-on-O-benzyloxim,
1-(2',3'-Dihydrobenzofuran-2'-yl)-2-(1,2,4-triazol-1-yl)-4,4-dimethylpentan-3-on-O-allyloxim,
1-(2',3'-Dihydrobenzofuran-2'-yl)-2-(1,2,4-triazol-1-yl)-4,4-dimethylpentan-3-on-O-propargyloxim,
1-(2',3'-Dihydrobenzofuran-2'-yl)-2-(1,2,4-triazol-1-yl)-4,4-dimethylpentan-3-on-O-(4-fluorbenzyl)-oxim,
1-(2',3'-Dihydrobenzofuran-2'-yl)-2-(1,2,4-triazol-1-yl)-4,4-dimethylpentan-3-on-O-(4-chlorbenzyl)-oxim,
1-(2',3'-Dihydrobenzofuran-2'-yl)-2-(1,2,4-triazol-1-yl)-4,4-dimethylpentan-3-on-O-acetyloxim,
1-(2',3'-Dihydrobenzofuran-2'-yl)-2-(1,2,4-triazol-1-yl)-4,4-dimethylpentan-3-on-O-propionyloxim,
1-(2',3'-Dihydrobenzofuran-2'-yl)-2-(1,2,4-triazol-1-yl)-4,4-dimethylpentan-3-on-O-chloracetyloxim,
1-(2',3'-Dihydrobenzofuran-2'-yl)-2-(1,2,4-triazol-1-yl)-4,4-dimethylpentan-3-on-O-benzoyloxim,
1-(2',3'-Dihydrobenzofuran-2'-yl)-2-(1,2,4-triazol-1-yl)-4,4-dimethylpentan-3-on-O-acetoacetyloxim,
1-(2',3'-Dihydro-3'-methylbenzofuran-2'-yl)-2-(1,2,4-triazol-1-yl)-4,4-dimethylpentan-3-on-oxim,
1-(2',3'-Dihydro-3'-methylbenzofuran-2'-yl)-2-(1,2,4-triazol-1-yl)-4,4-dimethylpentan-3-on-O-allyloxim,
1-(2',3'-Dihydro-3'-methylbenzofuran-2'-yl)-2-(1,2,4-triazol-1-yl)-4,4-dimethylpentan-3-on-O-propionyloxim,
1-(2',3'-Dihydro-5'-methylbenzofuran-2'-yl)-2-(1,2,4-triazol-1-yl)-4,4-dimethylpentan-3-on-oxim,
1-(2',3'-Dihydro-5'-methylbenzofuran-2'-yl)-2-(1,2,4-triazol-1-yl)-4,4-dimethylpentan-3-on-O-allyloxim,
1-(2',3'-Dihydro-5'-methylbenzofuran-2'-yl)-2-(1,2,4-triazol-1-yl)-4,4-dimethylpentan-3-on-O-acetyloxim,
1-(2',3'-Dihydro-5'-methylbenzofuran-2'-yl)-2-(1,2,4-triazol-1-yl)-4,4-dimethylpentan-3-on-O-propionyloxim,
1-(2',3'-Dihydro-5'-fluorbenzofuran-2'-yl)-2-(1,2,4-triazol-1-yl)-4,4-dimethylpentan-3-on-oxim,
1-(2',3'-Dihydro-5'-fluorbenzofuran-2'-yl)-2-(1,2,4-triazol-1-yl)-4,4-dimethylpentan-3-on-O-(4-chlorbutyl)-oxim,
1-(2',3'-Dihydro-5'-fluorbenzofuran-2'-yl)-2-(1,2,4-triazol-1-yl)-4,4-dimethylpentan-3-on-O-allyloxim,
1-(2',3'-Dihydro-5'-fluorbenzofuran-2'-yl)-2-(1,2,4-triazol-1-yl)-4,4-dimethylpentan-3-on-O-chloracetyloxim,
1-(2',3'-Dihydro-5'-fluorbenzofuran-2'-yl)-2-(1,2,4-triazol-1-yl)-4,4-dimethylpentan-3-on-O-propionyloxim,
1-(2',3'-Dihydro-5'-tert.-butylbenzofuran-2'-yl)-2-(1,2,4-triazol-1-yl)-4,4-dimethylpentan-3-oxim,
1-(2',3'-Dihydro-5'-tert.-butylbenzofuran-2'-yl)-2-(1,2,4-triazol-1-yl)-4,4-dimethylpentan-3-on-O-methyloxim,
1-(2',3'-Dihydro-5'-tert.-butylbenzofuran-2'-yl)-2-(1,2,4-triazol-1-yl)-4,4-dimethylpentan-3-on-O-allyloxim,
1-(2',3'-Dihydro-5'-tert.-butylbenzofuran-2'-yl)-2-(1,2,4-triazol-1-yl)-4,4-dimethylpentan-3-on-O-benzyloxim,
1-(2',3'-Dihydro-5'-tert.-butylbenzofuran-2'-yl)-2-(1,2,4-triazol-1-yl)-4,4-dimethylpentan-3-on-O-acetyloxim,
1-(2'-3'-Dihydro-5'-tert.-butylbenzofuran-2'-yl)-2-(1,2,4-triazol-1-yl)-4,4-dimethylpentan-3-on-O-chloracetyloxim,
1-(2'-3'-Dihydro-5'-tert.-butylbenzofuran-2'-yl)-2-(1,2,4-triazol-1-yl)-4,4-dimethylpentan-3-on-O-propionyloxim,
1-(2'-3'-Dihydro-5',7'-dimethylbenzofuran-2'-yl)-2-(1,2,4-triazol-1-yl)-4,4-dimethylpentan-3-on-oxim,
1-(2',3'-Dihydro-5'-chlorbenzofuran-2'-yl)-2-(1,2,4-triazol-1-yl)-4,4-dimethylpentan-3-on-oxim,
1-(2',3'-Dihydro-5'-chlorbenzofuran-2'-yl)-2-(1,2,4-triazol-1-yl)-4,4-dimethylpentan-3-on-O-allyloxim,
1-(2',3'-Dihydro-5'-chlorbenzofuran-2'-yl)-2-(1,2,4-triazol-1-yl)-4,4-dimethylpentan-3-on-O-propionyloxim,
1-(2',3'-Dihydro-5',7'-dichlorbenzofuran-2'-yl)-2-(1,2,4-triazol-1-yl)-4,4-dimethylpentan-3-on-oxim,
1-(2',3'-Dihydro-5',7'-dichlorbenzofuran-2'-yl)-2-(1,2,4-triazol-1-yl)-4,4-dimethylpentan-3-on-O-methyloxim,
1-(2',3'-Dihydro-5',7'-dichlorbenzofuran-2'-yl)-2-(1,2,4-triazol-1-yl)-4,4-dimethylpentan-3-on-O-allyloxim,
1-(2',3'-Dihydro-5',7'-dichlorbenzofuran-2'-yl)-2-(1,2,4-triazol-1-yl)-4,4-dimethylpentan-3-on-O-benzyloxim,
1-(2',3'-Dihydro-5',7'-dichlorbenzofuran-2'-yl)-2-(1,2,4-triazol-1-yl)-4,4-dimethylpentan-3-on-O-acetyloxim,
1-(2',3'-Dihydro-5',7'-dichlorbenzofuran-2'-yl)-2-(1,2,4-triazol-1-yl)-4,4-dimethylpentan-3-on-O-methoxyacetyloxim,
1-(2',3'-Dihydro-5',7'-dichlorbenzofuran-2'-yl)-2-(1,2,4-triazol-1-yl)-4,4-dimethylpentan-3-on-O-propionaloxim,
1-(2',3'-Dihydro-3'-methyl-5',7'-dichlorbenzofuran-2'-yl)-2-(1,2,4-triazol-1-yl)-4,4-dimethylpentan-3-on-oxim,
1-(2',3'-Dihydro-3'-methyl-5',7'-dichlorbenzofuran-2'-yl)-2-(1,2,4-triazol-1-yl)-4,4-dimethylpentan-3-on-O-propionaloxim,
1-(2',3'-Dihydro-3'-methyl-5',7'-dichlorbenzofuran-2'-yl)-2-(1,2,4-triazol-1-yl)-4,4-dimethylpentan-3-on-O-benzoyloxim,
1-(2',3'-Dihydro-4',6'-dichlorbenzofuran-2'-yl)-2-(1,2,4-triazol-1-yl)-4,4-dimethylpentan-3-on-oxim,
1-(2',3'-Dihydro-4',6'-dichlorbenzofuran-2'-yl)-2-(1,2,4-triazol-1-yl)-4,4-dimethylpentan-3-on-O-propionyloxim,
1-(2',3'-Dihydro-5'-brom-7'-chlorbenzofuran-2'-yl)-2-(1,2,4-triazol-1-yl)-4,4-dimethylpentan-3-on-oxim,
1-(2',3'-Dihydro-5'-brom-7'-chlorbenzofuran-2'-yl)-2-(1,2,4-triazol-1-yl)-4,4-dimethylpentan-3-on-O-allyloxim,
1-(2',3'-Dihydro-5'-brom-7'-chlorbenzofuran-2'-yl)-2-(1,2,4-triazol-1-yl)-4,4-dimethylpentan-3-on-O-(4-trifluormethylbenzyl)-oxim,
1-(2',3'-Dihydro-5'-brom-7'-chlorbenzofuran-2'-yl)-2-(1,2,4-triazol-1-yl)-4,4-dimethylpentan-3-on-O-acetyloxim,
1-(2',3'-Dihydro-5'-chlor-7'-methoxybenzofuran-2'-yl)-2-(1,2,4-triazol-1-yl)-4,4-dimethylpentan-3-on-oxim,
1-(2',3'-Dihydro-5'-chlor-7'-methylbenzofuran-2'-yl)-2-(1,2,4-triazol-1-yl)-4,4-dimethylpentan-3-on-O-acetyloxim,
1-(2',3'-Dihydro-5'-chlor-7'-methylbenzofuran-2'-yl)-2-(1,2,4-triazol-1-yl)-4,4-dimethylpentan-3-on-O-propionyloxim,
1-(2',3'-Dihydro-5'-methoxybenzofuran-2'-yl)-2-(1,2,4-triazol-1-yl)-4,4-dimethylpentan-3-on-oxim,
1-(2',3'-Dihydro-5'-methoxybenzofuran-2'-yl)-2-(1,2,4-triazol-1-yl)-4,4-dimethylpentan-3-on-O-methyloxim,
1-(2',3'-Dihydro-5'-methoxybenzofuran-2'-yl)-2-(1,2,4-triazol-1-yl)-4,4-dimethylpentan-3-on-O-ethyloxim,
1-(2',3'-Dihydro-5'-methoxybenzofuran-2'-yl)-2-(1,2,4-triazol-1-yl)-4,4-dimethylpentan-3-on-O-allyloxim,
1-(2',3'-Dihydro-5'-methoxybenzofuran-2'-yl)-2-(1,2,4-triazol-1-yl)-4,4-dimethylpentan-3-on-O-propargyloxim,
1-(2',3'-Dihydro-5'-methoxybenzofuran-2'-yl)-2-(1,2,4-triazol-1-yl)-4,4-dimethylpentan-3-on-O-benzyloxim,
1-(2',3'-Dihydro-5'-methoxybenzofuran-2'-yl)-2-(1,2,4-triazol-1-yl)-4,4-dimethylpentan-3-on-O-acetyloxim,
1-(2',3'-Dihydro-5'-methoxybenzofuran-2'-yl)-2-(1,2,4-triazol-1-yl)-4,4-dimethylpentan-3-on-O-propionyloxim,
1-(2',3'-Dihydro-5'-methoxybenzofuran-2'-yl)-2-(1,2,4-triazol-1-yl)-4,4-dimethylpentan-3-on-O-benzoyloxim,
1-(2',3'-Dihydro-5'-methyl-5'-methoxybenzofuran-2'-yl)-2-(1,2,4-triazol-1-yl)-4,4-dimethylpentan-3-on-oxim,
1-(2',3'-Dihydro-nitrobenzofuran-2'-yl)-2-(1,2,4-triazol-1-yl)-4,4-dimethylpentan-3-on-oxim,
1-(2',3'-Dihydro-5'-ethoxybenzofuran-2'-yl)-2-(1,2,4-triazol-1-yl)-4,4-dimethylpentan-3-on-oxim,
1-(2',3'-Dihydro-5'-ethoxybenzofuran-2'-yl)-2-(1,2,4-triazol-1-yl)-4,4-dimethylpentan-3-on-O-propionyloxim,
1-(2',3'-Dihydro-5'-trifluormethylbenzofuran-2'-yl)-2-(1,2,4-triazol-1-yl)-4,4-dimethylpentan-3-on-oxim,
1-(2',3'-Dihydro-4',5',7'-trichlorbenzofuran-2'-yl)-2-(1,2,4-triazol-1-yl)-4,4-dimethylpentan-3-on-oxim.

Zur Erläuterung der Erfindung wurden die in den Beispielen und in der Tabelle 1 im einzelnen genannten Verbindungen hergestellt und, soweit angegeben, auf ihre biologische Wirksamkeit untersucht.

*Beispiel 1:*

Zu einer Lösung von 6 g (0,086 Mol) Hydroxylaminhydrochlorid und 6 g (0,073 Mol) Natriumacetat in 60 ml Wasser wurde bei 60°C eine Lösung von 9,3 g (0,0283 Mol) 1-(2',3'-Dihydro-5'-methoxybenzofuran-2'-yl)-2-(1,2,4-triazol-1-yl)-4,4-dimethylpentan-3-on in 90 ml Ethanol schnell zugesetzt.

Nach 8tägigem Rühren bei 60°C wurde eingeengt, der Rückstand nacheinander mit 200 ml Essigester und 100 ml einer 5%igen wässrigen Natriumhydrogencarbonat-Lösung versetzt und 10 min gerührt. Die organische Schicht wurde über $Na_2SO_4$ getrocknet und im Vakuum eingeengt. Der verbleibende kristalline, farblose Rückstand wurde mit 20 ml Ether versetzt, eine Stunde bei 5°C stehengelassen, abgesaugt, mit Petrolether gewaschen und getrocknet. Es wurden 6,1 g (62,6% d.Th.) 1-(2',3'-Dihydro-5'-methoxybenzofuran-2'-yl)-2-(1,2,4-triazol-1-yl)-4,4-dimethylpentan-3-on-oxim vom Schmelzpunkt 157 bis 159°C erhalten.

*Beispiel 2:*

Eine Mischung aus 8,6 g (0,025 mol) 1-(2',3'-Dihydro-5'-methoxybenzofuran-2'-yl)-2-(1,2,4-triazol-1-yl)-4,4-dimethylpentan-3-on-oxim, 0,5 g Imidazol und 50 ml Propionsäureanhydrid wurde 10 h bei 70°C gerührt und anschliessend im Vakuum eingeengt. Der Rückstand wurde in 150 ml Ether gelöst, 20 min mit 50 ml einer 5%igen Natriumhydrogencarbonat-Lösung gerührt, die überstehende organische Phase über Natriumsulfat getrocknet und schliesslich bei bis zu 70°C/0,2 mbar eingeengt. Man erhält 7,6 g (76% d.Th.) 1-(2',3'-Dihydro-5'-methoxybenzofuran-2'-yl)-2-(1,2,4-triazol-1-yl)-4,4-dimethylpentan-3-on-O-propionyloxim als hellgelbes Harz.

IR (Film): 3120, 2965, 1735, 1470, 1360, 1268, 1220, 1130, 1100, 868, 747, 672 $cm^{-1}$.

Tabelle 1: (Bedeutung der Substituenten wie Formel I)

| Nr. | X | R | $R^1$ | Fp/° C |
|---|---|---|---|---|
| 3 | H | H | H | 153-159 |
| 4 | 5-(tert.-$C_4H_9$) | H | H | 224-228 |
| 5 | 5,7-$Cl_2$ | H | H | 134-138 |
| 6 | 5,7-$Cl_2$ | $-CH_3$ | H | 171-186 |
| 7 | 5-Br, 7-Cl | H | H | 174-180 |

Die neuen Verbindungen können praktisch alle Entwicklungsstadien einer Pflanze verschiedenartig beeinflussen und werden deshalb als Wachstumsregulatoren eingesetzt.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z.B. Sonnenschein, Dauer, Durchschnittstemperatur, Niederschlagsmenge,

e) von der Bodenbeschaffenheit (einschliesslich Düngung),

f) von der Formulierung bzw. Anwendungsform des Wirkstoffs und schliesslich

g) von den angewendeten Konzentrationen der aktiven Substanz.

Aus der Reihe der verschiedenartigen Anwendungsmöglichkeiten der erfindungsgemässen Pflanzenwachstumsregulatoren im Pflanzenanbau, in der Landwirtschaft und im Gartenbau, werden einige nachstehend erwähnt.

A. Mit den erfindungsgemäss verwendbaren Verbindungen lässt sich das vegetative Wachstum der Pflanzen stark hemmen, was sich insbesondere in einer Reduzierung des Längenwachstums äussert. Die behandelten Pflanzen weisen demgemäss einen gedrungenen Wuchs auf; ausserdem ist eine dunklere Blattfärbung zu beobachten.

Als vorteilhaft für die Praxis erweist sich z.B. die Verringerung des Grabsbewuchses an Strassenrändern, Kanalböschungen und auf Rasenflächen wie Park-, Sport- und Obstanlagen, Zierrasen und Flugplätzen, so dass der arbeits- und kostenaufwendige Rasenschnitt reduziert werden kann.

Von wirtschaftlichem Interesse ist auch die Erhöhung der Standfestigkeit von lageranfälligen

Kulturen wie Getreide, Mais, Sonnenblumen und Soja. Die dabei verursachte Halmverkürzung und Halmverstärkung verringern oder beseitigen die Gefahr des «Lagerns» (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern lässt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemässen Verbindungen im Herbst zwar gut bestockt werden, aber nicht zu üppig in den Winter hineingehen. Dadurch kann der erhöhten Frostempfindlichkeit und – wegen der relativ geringen Blatt- bzw. Pflanzenmasse – dem Befall mit verschiedenen Krankheiten (z.B. Pilzkrankheit) vorgebeugt werden. Die Hemmung des vegetativen Wachstums ermöglicht ausserdem bei vielen Kulturpflanzen eine dichtere Bepflanzung des Bodens, so dass ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum grösserer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluss zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schliesslich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfrallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heisst die Förderung der Ausbildung von Trenngewebe zwischen Frucht- bzw. Blatt- und Sprossteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemässen Wirkstoffe können den Kulturpflanzen sowohl vom Samen her (als Saatgutbeizmittel) als auch über den Boden, d.h. durch die Wurzel sowie – besonders bevorzugt – durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha, bevorzugt 0.25 bis 3 kg/ha, als ausreichend zu betrachten.

Die erfindungsgemässen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemässen Verbindungen in wässeriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Hinweise für eine geeignete Formulierung der erfindungsgemässen Stoffe können beispielsweise den Angaben in der DE-OS 3025879 bzw. der EP-OS 0044407 entnommen werden.

Die erfindungsgemässen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrösserung des Wirkungsspektrums. Bei einer Anzahl solcher Wachstumsregulatormischungen treten auch synergistische Effekte auf, d.h. die Wirksamkeit des Kombinationsproduktes ist grösser als die addierten Wirksamkeiten der Einzelkomponenten.

Fungizide, die mit den erfindungsgemässen Verbindungen kombiniert werden können, sind beispielsweise Dithiocarbamate und deren Derivate, wie

Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfid,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2))-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäureamid,
D,L-Methyl-N-(2,6-dimethyl-phenyl)-N-furyl-(2)-alaninat,
D,L-N-(2,6-Dimethyl-phenyl)-N-(2'-methoxyacetyl)-alanin-methylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methyl-isocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In den nachfolgenden Beispielen A und B wird die Wirkung der erfindungsgemäss verwendbaren Stoffe als Pflanzenwachstumsregulatoren dargestellt, ohne die Möglichkeiten weiterer Anwendungen auszuschliessen.

*Anwendungsbeispiele*

Zur Darstellung der wachstumsregulierenden Eigenschaft der erfindungsgemässen Stoffe wurden Testpflanzen auf ausreichend mit Nährstoffen versorgtem Torfkultursubstrat in Kunststoffgefässen von ca. 12,5 cm Durchmesser angezogen.

Im Vorlaufverfahren wurden die Stoffe in wässriger Aufbereitung am Tage der Einsaat auf das Saatbeet gegossen.

Im Nachauflaufverfahren wurden die Stoffe als wässrige Zubereitung auf die Pflanzen gesprüht. Die wachstumsregulierende Wirkung wurde bei Versuchsende durch Vergleich der Wuchshöhe der behandelten Pflanzen zur Wuchshöhe der unbehandelten Pflanzen ermittelt.

Als Vergleichssubstanz des Standes der Technik wurde das eingangs erwähnte Chlorcholinchlorid (CCC) gewählt.

*1. Sonnenblumen, Sorte «Sobrid»*
*Nachauflaufverfahren*

| Nr. d. chem. Beispiele | Aufwandmengen mg WS/Gefäss | Wuchshöhen rel. |
|---|---|---|
| unbehandelt | — | 100 |
| CCC | 1,5 | 93,7 |
| | 6,0 | 92,0 |
| 1 | 1,5 | 94,6 |
| | 6,0 | 87,8 |
| 3 | 1,5 | 73,6 |
| | 6,0 | 70,3 |
| 9 | 1,5 | 88,5 |
| | 6,0 | 88,5 |
| 12 | 1,5 | 94,5 |
| | 6,0 | 88,6 |
| 14 | 1,5 | 96,5 |
| | 6,0 | 90,6 |

*2. Sommerraps, Sorte «Petranova»*
*Vorauflaufverfahren*

| Nr. d. chem. Beispiele | Aufwandmengen mg WS/Gefäss | Wuchshöhen rel. |
|---|---|---|
| unbehandelt | — | 100 |
| CCC | 3 | 95,1 |
| | 12 | 90,2 |
| 1 | 3 | 101,4 |
| | 12 | 96,0 |
| 3 | 3 | 60,0 |
| | 12 | 40,0 |
| 9 | 3 | 100,0 |
| | 12 | 88,6 |
| 14 | 3 | 95,1 |
| | 12 | 82,9 |

*3. Sommerraps, Sorte «Petranova»*
*Nachauflaufverfahren*

| Nr. d. chem. Beispiele | Aufwandmengen mg WS/Gefäss | Wuchshöhen rel. |
|---|---|---|
| unbehandelt | — | 100 |
| CCC | 1,5 | 91,3 |
| | 6,0 | 84,5 |
| 1 | 1,5 | 91,1 |
| | 6,0 | 85,5 |
| 3 | 1,5 | 56,9 |
| | 6,0 | 51,0 |
| 9 | 1,5 | 74,9 |
| | 6,0 | 62,8 |
| 12 | 1,5 | 70,8 |
| | 6,0 | 61,6 |
| 14 | 1,5 | 61,6 |
| | 6,0 | 52,5 |

**Patentansprüche**

1. Triazolylalkyl-2,3-dihydrobenzofuran-oxime der Formel I

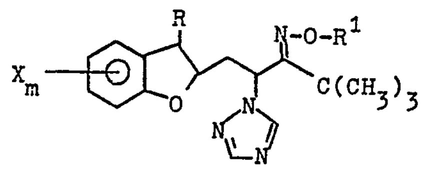

(I)

in der

R ein Wasserstoffatom oder einen Alkylrest mit bis zu 4 C-Atomen,

X ein Wasserstoff-, Fluor-, Chlor-, Brom- oder Iodatom, einen Nitro-, Trifluormethyl- oder einen Alkylrest mit bis zu 4 C-Atomen, einen Alkoxyrest mit bis zu 2 C-Atomen, einen Alkenylrest mit 3 bis 4 C-Atomen, einen Phenyl- oder Phenoxyrest und

m eine ganze Zahl von 1 bis 4 bedeutet, wobei die einzelnen Gruppen X gleich oder verschieden sind, wenn m grösser als 1 ist, in der ferner

$R^1$ ein Wasserstoffatom, einen gegebenenfalls chlorsubstituierten Alkylrest mit 1 bis 6 C-Atomen der einen gegebenenfalls chlorsubstituierten Alkenylrest mit 3 bis 5 C-Atomen, einen Alkinylrest mit 3 bis 4 C-Atomen oder einen Benzylrest bedeutet, wobei der Benzylrest durch ein Fluor-, Chlor- oder Bromatom, eine Nitrogruppe, einen Trifluormethyl- oder einen Alkylrest mit bis zu 4 C-Atomen substituiert sein kann, oder in der $R^1$ einen $-CO-R^2-$Rest bedeutet, wobei

$R^2$ Methyl, Ethyl, n-Propyl, Isopropyl, Chlormethyl, Chlorpropyl, Methoxymethyl, Acetonyl ($CH_3-COCH_2-$), Vinyl und Propenyl bedeutet.

2. Die Umsetzungsprodukte aus Verbindungen I gemäss Anspruch 1 und einer Säure oder einem komplexbildenden Metallsalz.

3. Triazolyl-2,3-dihydrobenzofuran-oxime I gemäss Anspruch 1, ausgewählt aus der Gruppe bestehend aus 1-(2',3'-Dihydrobenzofuran-2'-yl)-2-(1,2,4-triazolyl-(1))-4,4-dimethyl-pentan-3-on-oxim, 1-(2',3'-Dihydrobenzo-furan-2'-yl)-2-(1,2,4-triazolyl-(1))-4,4-dimethylpentan-3-on-O-propionyloxim, 1-(2',3'-Dihydrobenzofuran-2'-yl)-2-(1,2,4-triazolyl-(1))-4,4-dimethylpentan-3-on-O-allyloxim, 1-(3'-Methyl-5',7'-dichlor-2',3'-dihydrobenzofuran-2'-yl)-2-(1,2,4-triazolyl-(1))-4,4-dimethylpentan-3-on-oxim, 1-(3'-Methyl-5',7'-dichlor-2',3'-dihydrobenzofuran-2'-yl)-2-(1,2,4-triazolyl-(1))-4,4-dimethylpentan-3-on-O-propionyloxim, 1-(5',7'-Dichlor-2',3'-dihydrobenzofuran-2'-yl)-2-(1,2,4-triazolyl-(1))-4,4-dimethylpentan-3-on-oxim, 1-(5'-Brom-7'-chlor-2',3'-dihydrobenzofuran-2'-yl)-2-(1,2,4-triazolyl-(1))-4,4-dimethylpentan-3-on-oxim, 1-(5'-Methoxy-2',3'-dihydrobenzofuran-2'-yl)-2-(1,2,4-triazolyl-(1))-4,4-dimethylpentan-3-on-oxim, 1-(5'-Methoxy-2',3'-dihydrobenzofuran-2'-yl)-2-(1,2,4-triazolyl-(1))-4,4-dimethylpentan-3-on-O-propionyloxim, 1-(5'-tert.-Butyl-2',3'-dihydrobenzofuran-2'-yl)-2-(1,2,4-triazolyl-(1))-4,4-dimethylpentan-3-on-oxim und 1-(5'-tert.-Butyl-2',3'-dihydrobenzofuran-2'-yl)-2-(1,2,4-triazolyl-(1))-4,4-dimethylpentan-3-on-O-propionyloxim.

4. Verfahren zur Herstellung von Triazolyl-2,3-dihydrobenzofuran-oximen der Formel I gemäss Anspruch 1, dadurch gekennzeichnet, dass man Triazolylalkyl-2,3-dihydrobenzofuranone der Formel II

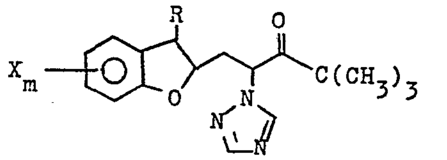 (II)

in der

X, R und m die im Anspruch 1 angegebenen Bedeutungen haben, mit Hydroxylamin-Derivaten der Formel III

$$H_2N-O-R^1 \qquad (III)$$

in der

$R^1$ die in Anspruch 1 angegebene Bedeutung hat, in Gegenwart eines basischen oder sauren Katalysators und gegebenenfalls in Gegenwart eines Verdünnungsmittels umsetzt.

5. Verfahren zur Herstellung von Triazolyl-2,3-dihydrobenzofuran-oximen der Formel I gemäss Anspruch 1, dadurch gekennzeichnet, dass man Triazolyl-2,3-dihydrobenzofuran-oxime der Formel I ($R^1$=H), mit einem entsprechenden Alkyl-, Alkenyl- oder Benzylhalogenid $R^1$-Hal, in der $R^1$ die im Anspruch 1 angegebene Bedeutung hat, einen Säurechlorid oder -anhydrid der Formel $R^2-CO-Hal$ bzw. $R^2-CO-O-CO-R^2$, wobei $R^2$ die in Anspruch 1 angegebene Bedeutung hat, in Gegenwart einer anorganischen oder organischen Base, gegebenenfalls unter Zusatz eines Reaktionsbeschleunigers und/oder Phasentransferkatalysators und gegebenenfalls in Gegenwart eines Verdünnungsmittels umsetzt.

6. Mittel zur Regulierung des Pflanzenwachstums, enthaltend eine oder mehrere der Verbindungen gemäss Anspruch 1.

7. Mittel zur Regulierung des Pflanzenwachstums, enthaltend eine oder mehrere der Verbindungen gemäss Anspruch 1 und einen flüssigen oder festen Trägerstoff sowie gegebenenfalls eine oder mehrere oberflächenaktive Mittel.

8. Verwendung von Verbindungen gemäss Anspruch 1 zur Regulierung des Pflanzenwachstums.

9. Verfahren zur Regulierung des Pflanzenwachstums, dadurch gekennzeichnet, dass man eine oder mehrere Verbindungen gemäss Anspruch 1 auf Pflanzen oder deren Lebensraum einwirken lässt.

10. Verfahren zur Herstellung von das Pflanzenwachstum regulierenden Mitteln, dadurch gekennzeichnet, dass man eine oder mehrere der Verbindungen gemäss Anspruch 1 mit festen oder flüssigen Trägerstoffen sowie gegebenenfalls einem oder mehreren oberflächenaktiven Mitteln mischt.

## Claims

1. A triazolylalkyl-2,3-dihydrobenzofuran oxime of the formula I

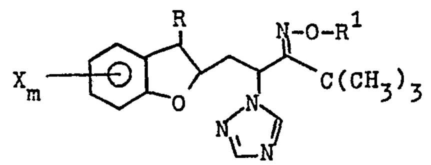 (I),

where

R is hydrogen or alkyl of up to 4 carbon atoms,

X is hydrogen, fluorine, chlorine, bromine, iodine, nitro, trifluoromethyl, alkyl of up to 4 carbon atoms, alkoxy of up to 2 carbon atoms, alkenyl of 3 or 4 carbon atoms, phenyl or phenoxy,

m is an integer from 1 to 4, the individual groups X being identical or different if m is larger than 1,

$R^1$ is hydrogen, an optionally chlorine-substituted alkyl of 1 to 6 carbon atoms, an optionally chlorine-substituted alkenyl of 3 to 5 carbon atoms, alkynyl of 3 or 4 carbon atoms or benzyl, the benzyl radical being optionally substituted by fluorine, chlorine, bromine, nitro, trifluoromethyl or alkyl of up to 4 carbon atoms, or

$R^1$ is $-CO-R^2$, where $R^2$ is methyl, ethyl, n-propyl, isopropyl, chloromethyl, chloropropyl, methoxymethyl, acetonyl, ($CH_3-COCH_2-$), vinyl or propenyl.

2. The product obtained by reacting a compound I as claimed in claim 1 with an acid or a complex-forming metal salt.

3. A triazolyl-2,3-dihydrobenzofuran oxime I as claimed in claim 1, selected from the group consisting of

1-(2',3'-dihydrobenzofuran-2'-yl)-2-(1,2,4-triazolyl-(1))-4,4-dimethyl-pentan-3-on-oxime, 1-(2',3'-dihydrobenzofuran-2'-yl)-2-(1,2,4-triazolyl-(1))-4,4-dimethylpentan-3-one-O-propionyloxime, 1-(2',3'-dihydrobenzofuran-2'-yl)-2-(1,2,4-triazolyl-(1))-4,4-dimethylpentan-3-one-O-allyl-oxime, 1-(3'-methyl-5',7'-dichloro-2',3'-dihydrobenzofuran-2'-yl)-2-(1,2,4-triazolyl-(1))-4,4-dimethylpentan-3-one-oxime, 1-(3'-methyl-5',7'-dichloro-2',3'-dihydrobenzofuran-2'-yl)-2-(1,2,4-triazolyl-(1))-4,4-dimethylpentan-3-one-O-propionyloxime, 1-(5',7'-dichloro-2',3'-dihydrobenzofuran-1'-yl)-2-(1,2,4-triazolyl-(1))-4,4-dimethylpentan-3-one-oxime, 1-(5'-bromo-7'-chloro-2',3'-dihydrobenzofuran-2'-yl)-2-(1,2,4-triazolyl-(1))-4,4-dimethylpentan-3-one-oxime, 1-(5'-methoxy-2',3'-dihydrobenzofuran-2'-yl)-2-(1,2,4-triazol-(1))-4,4-dimethylpentan-3-one-oxime, 1-(5'-methoxy-2',3'-dihydrobenzofuran-2'-yl)-2-(1,2,4-triazolyl-(1))-4,4-dimethylpentan-3-one-O-propionyloxime, 1-(5'-tert.-butyl-2',3'-dihydrobenzofuran-2'-yl)-2-(1,2,4-triazolyl-(1))-4,4-dimethylpentan-3-one-oxime and 1-(5'-tert.-butyl-2',3'-dihydrobenzofuran-2'-yl)-2-(1,2,4-triazolyl-(1))-4,4-dimethylpentan-3-one-O-propionyloxime.

4. A process for the preparation of a triazolyl-2,3-dihydrobenzofuran oxime of the formula I as claimed in claim 1, wherein a triazolylalkyl-2,3-dihydrobenzofuranone of the formula II

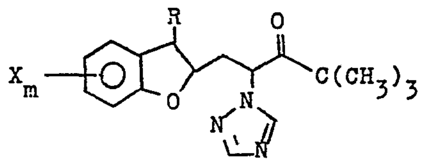 (II),

where X, R and m have the meanings given in claim 1, is reacted with a hydroxylamine derivative of the formula III

$$H_2N-O-R^1 \qquad (III),$$

where $R^1$ has the meanings given in claim 1, in the presence of a basic or acidic catalyst and in the presence or absence of a diluent.

5. A process for the preparation of a triazolyl-2,3-dihydrobenzofuran oxime of the formula I as claimed in claim 1, wherein a triazolyl-2,3-dihydrobenzofuran oxime of the formula I ($R^1$=H) is reacted with a corresponding alkyl, alkenyl or benzyl halide $R^1-Hal$, where $R^1$ has the meanings given in claim 1, or with an acid chloride of the formula $R^2-CO-Hal$ or an acid anhydride of the formula $R^2-CO-O-CO-R^2$, in which formulae $R^2$ has the meanings given in claim 1, in the presence of an inorganic or organic base, with or without the addition of a reaction accelerator and/or a phase transfer catalyst, and in the presence or absence of a diluent.

6. An agent for regulating plant growth, containing one or more compounds as claimed in claim 1.

7. An agent for regulating plant growth, containing one or more compounds as claimed in claim 1, a solid or liquid carrier and, if desired, one or more surfactants.

8. The use of a compound as claimed in claim 1 for regulating plant growth.

9. A method of regulating plant growth, wherein one or more compounds as claimed in claim 1 are allowed to act on the plants or their habitat.

10. A process for the production of an agent for regulating plant growth, wherein one or more compounds as claimed in claim 1 are mixed with a solid or liquid carrier and, if disired, one or more surfactants.

**Revendications**

1. Triazolylalkyl-2,3-dihydrobenzofuran-oximes de formule I

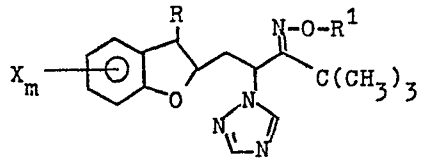 (I)

dans laquelle

R représente un atome d'hydrogène ou un reste alkyle ayant jusqu'à 4 atomes C,

X représente un atome d'hydrogène, de fluor, de chlore, de brome ou d'iode, un reste nitro, trifluorométhyle ou un reste alkyle ayant jusqu'à 4 atomes C, un reste alcoxy ayant jusqu'à 2 atomes C, un reste alcényle à 3 ou 4 atomes C, un reste phényle ou phénoxy et

m est un nombre entier de 1 à 4, les différents groupes X étant identiques ou différents si m est supérieur à 1, dans laquelle, en outre,

$R^1$ représente un atome d'hydrogène, un reste alkyle à 1 à 6 atomes C, éventuellement substitué par chlore, ou un reste alcényle à 3 à 5 atomes C, éventuellement substitué par chlore, un reste alcynyle à 3 ou 4 atomes C ou un reste benzyle, le reste benzyle pouvant être substitué par un atome de fluor, de chlore ou de brome, un groupe nitro, un reste trifluorométhyle ou un reste alkyle ayant jusqu'à 4 atomes C ou dans laquelle $R^1$ représente un reste $-CO-R^2$,

$R^2$ désignant méthyle, éthyle, n-propyle, isopropyle, chlorométhyle, chloropropyle, méthoxyméthyle, acétonyle ($CH_3-COCH_2-$), vinyle et propényle.

2. Les produits de réaction de composés I, selon la revendication 1, avec un acide ou un sel métallique susceptible de former des complexes.

3. Triazolyl-2,3-dihydrobenzofuran-oximes I, selon la revendication 1, choisies dans le groupe constitué de

1-(2',3'-dihydrobenzofuran-2'-yl)-2-(1,2,4-triazolyl-(1))-4,4-diméthylpentan-3-one-oxime, 1-(2',3'-dihydrobenzofuran-2'-yl)-2-(1,2,4-triazolyl-(1))-4,4-diméthylpentan-3-one-O-propionyloxime, 1-(2',3'-dihydrobenzofuran-2'-yl)-2-(1,2,4-triazolyl-(1))-4,4-diméthylpentan-3-one-O-allyloxime, 1-(3'-méthyl-5',7'-dichloro-2',3'-dihydrobenzofuran-2'-yl)-2-(1,2,4-triazolyl-(1))-4,4-diméthylpentan-3-one-oxime, 1-(3'-méthyl-5',7'-dichloro-2',3'-dihydrobenzofuran-2'-yl)-2-(1,2,4-triazolyl-(1))-4,4-diméthylpentan-3-one-O-propionyloxime, 1-(5',7'-dichloro-2',3'-dihydrobenzofuran-2'-yl)-2-(1,2,4-triazolyl-(1))-4,4-diméthylpentan-3-one-oxime, 1-(5'-bromo-7'-chloro-2',3'-dihydrobenzofuran-2'-yl)-2-(1,2,4-triazolyl-(1))-4,4-diméthylpentan-3-one-oxime, 1-(5'-méthoxy-2',3'-dihydrobenzofuran-2'-yl)-2-(1,2,4-triazolyl-(1))-4,4-diméthylpentan-3-one-oxime, 1-(5'-méthoxy-2',3'-dihydrobenzofuran-2'-yl)-2-(1,2,4-triazolyl-(1))-4,4-diméthylpentan-3-one-O-propionyloxime, 1-(5'-tert.-butyl-2',3'-dihydrobenzofuran-2'-yl)-2-(1,2,4-triazolyl-(1))-4,4-diméth-

ylpentan-3-one-oxime et 1-(5'-tert.-butyl-2',3'-dihydrobenzofuran-2'-yl)-2-(1,2,4-triazolyl-(1))-4,4-diméthylpentan-3-one-O-propionyl-oxime.

4. Procédé de préparation de triazolyl-2,3-dihydrobenzofuran-oximes de formule I, selon la revendication 1, caractérisé par le fait que l'on fait réagir, en présence d'un catalyseur basique ou acide et éventuellement en présence d'un diluant, des triazolylalkyl-2,3-dihydrobenzofuranes de formule II

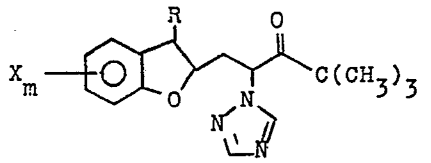

(II)

dans laquelle

X, R et m ont les significations données dans la revendication 1, avec des dérivés d'hydroxylamine de formule III

$$H_2N-O-R^1 \qquad (III)$$

dans laquelle

$R^1$ a la signification donnée dans la revendication 1.

5. Procédé de préparation de triazolyl-2,3-dihydrobenzofuran-oximes de formule I, selon la revendication 1, caractérisé par le fait que l'on fait réagir, en présence d'une base inorganique ou organique, éventuellement avec addition d'un sensibilisateur de réaction et/ou d'un catalyseur de transfert de phase et éventuellement en présence d'un diluant, des triazolyl-2,3-dihydrobenzofuran-oximes de formule I ($R^1=H$) avec un halogénure d'alkyle, d'alcényle ou de benzyle $R^1$-Hal approprié, $R^1$ ayant la signification donnée dans la revendication 1, un chlorure ou anhydride d'acide de formule $R^2-CO-Hal$ ou $R^2-CO-O-CO-R^2$, $R^2$ ayant la signification donnée dans la revendication 1.

6. Agents régulateurs de croissance des plantes, contenant un ou plusieurs composés selon la revendication 1.

7. Agents régulateurs de croissance des plantes, contenant un ou plusieurs composés selon la revendication 1 et un matériau support liquide ou solide, ainsi que, le cas échéant, un ou plusieurs surfactifs.

8. Utilisation de composés selon la revendication 1 pour la régulation de la croissance des plantes.

9. Procédé pour la régulation de la croissance des plantes, caractérisé par le fait que l'on fait agir sur les plantes ou leur biotope un ou plusieurs composés selon la revendication 1.

10. Procédé de préparation d'agents régulateurs de la croissance des plantes, caractérisé par le fait que l'on mélange un ou plusieurs composés selon la revendication 1 avec des matériaux supports solides ou liquides ainsi que, le cas échéant, un ou plusieurs surfactifs.